# EUROPEAN PATENT SPECIFICATION

(11) **EP 4 255 628 B1**
(45) Date of publication and mention of the grant of the patent: **29.10.2025**
(21) Application number: 21819863.8
(22) Date of filing: 29.11.2021
(51) Int. Cl.: B01L 1/02, C12M 1/34

(54) **FLUID CONTROL DEVICE WITH A CONTROL UNIT**
FLUIDSTEUERUNGSVORRICHTUNG MIT STEUEREINHEIT
DISPOSITIF DE RÉGULATION DE FLUIDE AVEC UNITÉ DE RÉGULATION

(30) Priority: 03.12.2020 LU 102254
(43) Date of publication of application: 11.10.2023
(73) Proprietor: cytena Bioprocess Solutions co., Ltd., Taipei City 110 (TW)
(72) Inventor: TSAI, Cheng-Han, Taipei (TW); KUAN, Da-Han, Taipei (TW); JIANG, Cheng-Yu, Taipei (TW)
(74) Representative: Grabovac, Dalibor
(86) International application number: PCT/EP2021/083388
(87) International publication number: WO 2022/117509

(56) References cited:
- WO-A1-2008/100033
- WO-A1-2018/232096
- WO-A1-2019/152920
- US-A1- 2018 272 346

## Description

The invention relates to a fluid control device comprising a control unit.

In a pharmaceutical process of biosimilar drugs, a cell line development process is required for most biopharmaceuticals. The cell line development is a method for selecting protein/antibodies, achieving a high yield of the protein/antibodies, and optimizing the quality thereof. However, the cell line development is on the other hand a very complicated, labor-intensive, and pricey process. Development time for most pharmaceutical factories is half a year.

A core tool required in the cell line development is a bioreactor for cell incubation. Starting from incubating single-cells in the process, the scale is continually expanded to a volume production scale through repeated transferring steps of selecting and scaling-up. Therefore, for different phases and different quantities of cells, bioreactors of different sizes ranging from a bioreactor at a microliter level to a large-scale bioreactor at a volume production level are required. However, there is a limitation on optimization of a cell incubating environment of a smaller-sized bioreactor. An ideal bioreactor generally needs to enable cells to continually grow in a suspension, dynamically monitor biosignals, and perform feedback control on dissolved oxygen content and a pH value. It is difficult to achieve the foregoing functions in a microliter-leveled bioreactor.

A conventional method in a cell incubation apparatus is to place cells in incubation well plates with different sizes (a 96-well plate and a 24-well plate) for static culture. However, in this way, the cells sink to the bottom of the well plates. Consequently, the cells grow in an inappropriate environment. The disadvantages of the static culture are as follows. 1. The growth environment of cells is limited to a 2D space. 2. Oxygen is dissolved into a culture solution from a gas-liquid interface above, and there is a distance between the cells and the oxygen. 3. Cell metabolites are continually accumulated around the cells; and the like.

In a bioreactor, there are many ways to achieve a culture environment of uniform mixture. Stirring or shaking is the most common manner to achieve the effect of uniform mixture. Nevertheless, because the liquid quantities are excessively small in a 96-well plate and a 24-well plate (Reynolds number is small), it is difficult to achieve the effect of uniform mixture in a conventional way. Therefore, for 96-well plate cell incubation and 24-well plate cell incubation, only static culture, at most a shaking manner, can be selected to achieve part of the mixture effect.

For most existing bioreactors, a manner of quick shake is used to achieve the effect of fluid mixture. However, the manner can achieve the effect of uniform mixture only in a 48-well plate and a 24-well plate, and there is no way to successfully conduct an experiment of uniform mixture in a 96-well plate. The reason is that the liquid quantity in the 96-well plate is excessively small and the Reynolds number is relatively small. As a result, the speed of quick shake needs to be greater than 1000 rpm to achieve the effect of mixture. Besides, during quick shake with such a speed, it is difficult to integrate optical sensors. Detection of optical signals cannot be performed during quick shake. That is why the well plate of the existing bioreactor can only have at most 48 wells.

WO 2018 / 189 398 A1 discloses a bioreactor comprising a control unit, a well for receiving a fluid sample comprising biological particles and a lid. The bioreactor has a stacked construction as the aforementioned components are arranged one above the other. The bioreactor has the disadvantage that it has a complex structure and that the operating flux of cultivation samples is low. WO 2019/152920 A1 discloses a cell culture system including an atmosphere controlled incubation chamber.

Therefore, the object of the invention is to provide a fluid control device with which the aforementioned disadvantage can be prevented.

The object is solved by a fluid control device comprising:
at least one treatment device having a chamber for receiving a processing device,
at least one processing device which is arranged in the chamber of the treatment device, and a control unit comprising
a pressure unit for providing positive and/or negative pressure,
at least one first control unit outlet being fluidically connected to the processing device comprising at least one receptacle for receiving a fluid sample,
at least one second control unit outlet being fluidically connected to the treatment device, and
a connection unit by means of which the pressure unit is fluidically connected with the first control unit outlet and/or with the second control unit outlet wherein
the control unit is adapted to control the processing of the fluid sample in the processing device by applying a positive or negative pressure provided by the pressure unit to the first control unit outlet by means of the connection unit and to control a physical state in the chamber of the treatment device.

The control unit has the advantage that it was recognized that the control unit and the treatment device can be separated from each other simplifying the structure of the treatment device. Additionally, it has be recognized that the same control unit can used for two functions, namely to control processing in the processing device and to control a physical state in the treatment device. It has been recognized that in contrary to the known embodiments the control unit is not only used for controlling the processing of the fluid sample arranged in the processing device but also for controlling the physical state in the chamber of the treatment device in which the processing device is arranged. In particular, it is possible to control the environment of the processing device by the control unit when the processing device is arranged within the chamber of the treatment device. Therefore, the productivity of cultivating biological particles is increased.

The treatment device and the processing device are not part of the control unit. The processing device can be arranged in the chamber of the treatment device. The processing device can be any kind of devices with which the fluid sample can be processed. In particular, the processing device can be a device with which fluid can be supplied or removed into the receptacle of the processing device in order to move and/or mix the fluid sample arranged in the receptacle. Such a device can comprise a lid and the receptacle and is described more in detail below. Alternatively, the processing device can be any kind of microfluidic devices in which a force has to be applied thereon in order to process the liquid within the microfluidic device. The microfluidic device can be a microfluidic chip.

The fluid sample depends on the fluid control device. If the fluid control device corresponds with a bioreactor, the fluid sample comprises a biological particle. The biological particle can be a cell or a microbe. The fluid sample can contain a liquid and at least one biological particle. The liquid can promote the growth of the biological particles, in particular cells or microbes, arranged in the liquid.

If the fluid control device corresponds with a chemical reactor, the liquid sample can comprise one or more chemical reagents. However, if the fluid control device comprises a microfluidic device as processing device, the fluid sample depends on the usage field of the microfluidic device.

The physical state in the chamber can be at least one physical parameter of the chamber like a chamber temperature or a chamber pressure or a gas humidity within the chamber or a gas composition within the chamber.

As mentioned above processing of fluid sample can comprise the supply or removal of fluid, in particular gas, into the receptacle of the processing and/or the mixing of the fluid sample and/or moving of the fluid sample and/or the agitation of biological particles of the fluid sample. Mixing of the fluid sample is understood as a process in which the components of the fluid sample are moved relative to each other in such a way that a new arrangement pattern is created. The processing device can perform at least one of the aforementioned processing steps and/or is controlled by applying positive or negative pressure to the first control unit outlet.

The control unit can be adapted to selectively apply positive or negative pressure to the first control unit outlet. The positive or negative pressure can be applied alternately to the first control unit outlet. Additionally or alternatively the negative or positive pressure ca be applied multiple times to the first control unit outlet. By applying the first control unit outlet with positive or negative pressure in the aforementioned manner, it is possible to secure that the fluid sample located in the processing unit is processed, in particular mixed.

A fluidic connection between two components exists when the fluid can flow from one component into the other component or vice versa.

The connection unit is a unit by means of which the pressure unit is fluidically connected or can be fluidically connected with the first control unit outlet and/or the second control unit outlet.

The first control unit outlet is an outlet through which a fluid, in particular gas, leaves the control unit or enters the control unit. Likewise, the second control unit outlet is an outlet through which a fluid, in particular gas, leaves the control unit or enters the control unit. The first control unit outlet can be part of a first tube in which the fluid, in particular gas, flows. Alternatively, the first control unit outlet can be part of a first connector with which a fluid connection can be established with the processing device. The second control unit outlet can be part of a second tube in which the fluid, in particular gas, flows. Alternatively, the second control unit outlet can be part of a second connector with which a fluid connection can be established with the treatment device.

According to an embodiment the control can be adapted to control the physical state in the chamber of the treatment device by fluidically connecting or disconnecting the pressure unit with the second control unit by means of the connection unit.

The pressure unit can comprise a pressurized gas tank having a positive pressure wherein the pressurized gas tank is fluidically connectable to the first control unit outlet and/or the second control unit outlet. A gas flows from the pressurized gas tank to the first control unit outlet and/or the second control unit outlet when the gas tank is fluidically connected to the first control unit and/or the second control unit.

Additionally or alternatively the pressure unit can comprise a vacuum gas tank having a negative tank wherein the vacuum gas tank is fluidically connectable to the first control unit outlet and/or the second control unit outlet. A gas flows from the first control unit outlet and/or the second control unit outlet to the vacuum gas tank when the first control unit outlet and/or the second control unit outlet is fluidically connected to the vacuum gas tank. A positive pressure gas is a gas having a pressure that is above the atmospheric pressure. A negative pressure gas is a gas having a pressure that is below the atmospheric pressure.

In addition, the pressure unit can comprise a gas mixer that is arranged upstream the pressurized gas tank. The gas mixer is used to mix at least two gases. Thereto, the gas mixer can comprise at least two inlets for a gas inlet. Ambient air or oxygen can be added via one inlet into the gas mixer. Carbon dioxide can be added via the other inlet into the gas mixer. Alternatively other gases can be added through at least one inlet into the gas mixer. The gas mixer can comprise an outlet for releasing the mixed gas.

The pressure unit can comprise a pump that is arranged upstream of the pressurized gas tank and/or that is adapted to increase pressure in the pressurized gas tank. The pump can be arranged downstream of the gas mixer. By providing the pump it is secured in a simple way that the pressure in the pressurized gas tank has a predetermined pressure. Additionally, a gas filter can be arranged upstream the pressurized tank for filtering gas. Thus, it can be prevented that e.g. dust particles are supplied to the treatment device and the processing device via the first control unit outlet and/or the second control unit outlet.

Furthermore, the pressure unit can comprise a further pump that is arranged downstream of the vacuum gas tank and/or that is adapted to decrease pressure in the vacuum gas tank. By providing the further pump the vacuum gas tank has a predetermined gas pressure. The pressure unit has a gas outlet through which the gas pumped by the further pump leaves the pressure unit. In particular, the gas outlet is arranged such that the gas pumped by the further pump is discharged to the environment. A further gas filter can be arranged downstream the vacuum gas tank.

The pressure unit can comprise a check valve. The check valve can be arranged fluidically between the pressurized gas tank and the first control unit outlet and/or second control unit outlet. This secures that only a gas flow from the pressurized gas tank to the first control unit outlet and/or the second control unit outlet occurs. Additionally, it secures that no gas can flow from the treatment device to the pressurized gas tank and/or from the processing device to the pressurized gas tank.

The control unit can supply the same gas to the treatment device and the processing device. In particular, the pressurized gas tank can supply the same gas to the treatment device and the processing device when the first control unit outlet and the second control unit outlet is fluidically connected to the pressurized gas tank. As the gas stored in the pressurized gas tank can be a gas mixture supporting the cultivation of the biological particles located in the processing device, the supply of the gas mixture into the processing device and the treatment device increases the productivity of cultivation of biological particles in an embodiment in which the fluid control device corresponds with a bioreactor.

According to an embodiment the connection unit can comprise at least one valve for selectively fluidical connecting the pressurized gas tank or the vacuum gas tank with the first control unit outlet. That means, the valve secures that either the pressurized gas tank or the vacuum gas tank is fluidically connected with the first control unit outlet. The valve can be arranged downstream of the pressurized gas tank.

Additionally, the connection unit comprises at least one further valve that in a first position fluidically connects the pressurized gas tank with, in particular the second control unit outlet and thus, the treatment device and in a second position separates the fluidically connection between the pressurized gas tank and, in particular the second control unit outlet and thus, the treatment device.

The control unit can comprise at least one controller for controlling the valve and/or the further valve. Thus, the controller can control whether the first control unit outlet and, thus, the processing device, is fluidically connected with the pressurized gas tank or with the vacuum gas tank. Additionally, the controller can control whether the pressurized gas tank is fluidically connected with the second control unit outlet and, thus, with the treatment device or not. The control can be based on sensor signals determined from one or more sensors. The sensors can detect at least one physical state in the chamber of the treatment device and/or of at least one physical state of the processing device.

In particular, the controller can control the valve based on a gas flow flowing through the first control unit outlet. In this matter the controller can control an opening size of an outlet of the valve based on the gas flow flowing through the first control unit outlet. That means, the valve is not only adapted to be fluidically connected with the first and/or second control unit outlet but it is also possible to control the gas flow through the valve.

The controller can additionally or alternatively control the valve based on a physical state, in particular pressure, in the chamber of the treatment device and on a physical state of the processing device, in particular a pressure within the processing device. The physical state of the treatment device and the physical state of the processing device can be detected by sensors.

At the end the controller can control whether the valve fluidically connects the first control unit outlet with the pressurized tank or the vacuum gas tank based on a physical state in the chamber of the treatment device and on a physical state of the processing device. That means, at least two physical state can be used for controlling the valve.

As mentioned above the controller can control the further valve. In particular, the controller can control the further valve based on a physical state of the chamber of the treatment device. The physical state can be a gas composition within the chamber. Alternatively and/or additionally, the controller can control the further valve based on a physical state of the processing device, in particular of a physical state of the fluid sample located in the processing device.

The controller controls the gas mixer based on a physical state, in particular a gas composition, of the chamber of the treatment device and/or of a physical state of the fluid sample in the processing device. As the processing device is fluidically connected with the treatment device a change of the physical state in the chamber, in particular the gas composition within the chamber, of the treatment device has a positive effect on the productivity of the biological particle cultivation.

An information about the fluid sample located in the processing device can be acquired by an acquisition unit. The acquisition unit can comprise an optical imaging apparatus like a camera that takes images of the processing device. Additionally, the acquisition unit can determine a physical state of the fluid sample located in the processing device based on the acquired information. The controller can control the gas mixer and/or the further valve based on the determined physical state of the fluid sample. The physical state can be a pH value of the liquid sample and/or an oxygen content of the liquid sample.

Additionally, the controller is adapted to control a humidifier arranged in the treatment device. Thus, it is possible that the controller controls the physical state, in particular gas humidity, in the chamber by controlling the humidification.

All the aforementioned control tasks can be performed by the same controller. Alternatively, it is possible that at least some tasks are performed by subcontrollers. In particular, the controller can comprise a first subcontroller for controlling whether the valve fluidically connects the first control unit outlet with the pressurized gas tank or with the vacuum gas tank and/or a second subcontroller for controlling the opening size of the valve outlet opening. Additionally or alternatively the controller can comprise a third subcontroller for controlling the further valve and/or the gas mixer and/or a fourth subcontroller for controlling the humidifier. The controller or the subcontroller can receive sensor signals as input signals.

According to an embodiment the control unit can comprise a housing. The housing can surround an inner space in which the pressurized gas tank and/or the vacuum gas tank and/or the pump and/or the further pump and/or the gas mixer and/or the valve and/or the further valve and/or the check valve is arranged. Thus, the pressure unit and the connection unit are arranged within the housing. This leads to a compact formed control unit comprising all necessary components to control the treatment device and the processing unit.

The housing can have at least one through hole for receiving a fluid connector of the treatment device and/or another through hole for receiving a further fluid connector of the processing device. In that case the fluid connector is connected with a tube of the control unit so that gas can flow from the control unit to the treatment device and vice versa. The further fluid connector is connected with a further tube of the control unit so that gas can flow form the control unit to the processing device and vice versa. The housing can be mechanically connected with the treatment device. The mechanical and/or fluidical connection can be in a releasable manner. Additionally, the housing can comprise an outlet that is arranged downstream the further pump and is fluidically connected with the further pump. Additionally, the housing can comprise at least two inlets that are arranged upstream the gas mixer and are fluidically connected with the gas mixer.

All of the aforementioned components can be fluidically connected with each other by tubes.

As mentioned before the control unit is adapted to control at least one physical state in the chamber of the treatment device and to control the processing of the fluid sample in the processing device. However, the same control unit can also be used to control a plurality of treatment devices and processing devices. This is done by that the control unit applies positive or negative pressure provided by the pressure unit on each of the first control unit outlets by means of the connection unit. The respective first control unit outlets can differ from each other in the applied pressure. Additionally, the control unit can control the physical state in the chambers of the treatment devices. The control unit can be adapted such that different physical states are controlled and/or the physical states differ from each other in the chamber of the treatment devices. Thus, it is not necessary anymore to provide each processing device with a control unit but the same, in particular merely one, control unit can be used for controlling a plurality of treatment devices and processing devices.

The control unit can comprise merely one pressure unit and/or several connection units. The number of connection units can correspond with the number of treatment devices and/or processing devices that can be fluidically connected with the control unit. Each of the connection units can comprise the same components and can have the structure as described above. The pressure unit can comprise the same components and have the same structure as described above.

In a particular embodiment the control unit can comprise a plurality of first control unit outlets and a plurality of second control unit outlets. Each of the plurality of first control unit outlets can be fluidically connected to one processing device. Additionally, each of the plurality of second control unit outlets can be fluidically connected to one treatment device. Each of the plurality of first control unit outlets is independent on the other first control unit outlets. Thus, a gas flow can occur via one or more of the first control unit outlets. Each of the plurality of second control unit outlets is independent on the other second control unit outlets. Thus, a gas flow can occur via one or more of the second control unit outlets.

The same pressurized gas tank can be connectable with each of the plurality of first control unit outlets and/or with each of the plurality of second control unit outlets. Additionally or alternatively the same vacuum gas tank can be connectable with each of the plurality of first control unit outlets.

As mentioned before the control unit can comprise a plurality of connection units so that the control unit can comprise a plurality of valves and a plurality of valves. Each of the valves is fluidically connected with one of the plurality of first control unit outlets and with the pressurized gas tank and the vacuum gas tank. Each of valves is fluidically connected with one of the plurality of second control unit outlets and with the pressurized gas tank.

In an embodiment in which the control unit can be fluidically connected with a plurality of treatment devices and/or processing devices the control unit can comprise one or more controllers. If the control unit comprises one controller, said controller controls the physical state in all treatment devices and the processing of the liquid sample in all processing devices. Alternatively, the control unit can comprise several controllers. In particular, the amount of controllers can correspond to the amount of connection units. The controllers can have subcontrollers as described above, respectively.

The pressure unit can have tubes enabling to fluidically connect the pressure unit with the treatment devices and/or processing devices.

The fluid control device comprises the control unit, at least one treatment device and at least one processing device which is arranged in the chamber of the treatment device. The processing device is fluidically connected with the first control unit outlet and the treatment device is connected with the second control unit outlet.

The fluid control device can correspond with a bioreactor. In such an embodiment the receptacle or receptacles of the processing device can be at microliter level. Alternatively, the receptacle or receptacles can have larger scale.

An advantageous fluid control device is achieved if the fluid control device comprises several treatment devices wherein a processing device can be arranged in the chamber of the respective treatment device. Such a fluid control device has the advantage that it is compact as the same control unit can be used for different treatment devices and/or processing devices.

The processing device can comprise at least one receptacle for receiving the fluid sample and a lid covering the receptacle. The lid can comprise at least one extension tube extending into the receptacle and can be fluidically connected with the first control unit outlet. The lid can comprise an inner space which is fluidically connected to the first control unit outlet and the receptacle. The processing device can have a plurality of receptacles. In particular, the processing device can comprise a multiwell plate. In said case the lid can have more than one extension tube. In particular, the lid can be formed that at least one extension tube extends in each of the receptacles.

The controller can control the valve such that a positive pressure provided by the pressurized gas tank is applied via the lid into the receptacle or a negative pressure provided by the vacuum gas tank is applied via the lid into the receptacle. By application of such a pressure the fluid sample can be aspirated into the extension tube or dispensed from the extension tube.

The controller controls the further valve such that a predetermined physical state is achieved in the chamber of the treatment device. The predetermined physical state can be the gas composition with the chamber. Additionally, the controller can control the humidifier such that the gas in the chamber has a predetermined humidity.

The treatment device can comprise a heating unit for heating the processing device /and or the chamber of the treatment device. The controller can control the physical state in the chamber, namely by controlling the heating unit in order to increase the chamber temperature. Alternatively, the controller can control the heating unit in order to increase the temperature of the liquid sample located in the processing unit.

The treatment device can comprise a housing surrounding the chamber, in which the processing device is arranged. Thus, the treatment device has a simple structure. The treatment device comprises a transparent housing part enabling the acquisition unit to acquire information about the fluid sample located in the processing device.

In the figures, the subject-matter of the invention is schematically shown, wherein identical or similarly acting elements are usually provided with the same reference signs.
FIG. 1 (A) is an exploded diagram of a processing device.
Fig. 1 (B) is a schematic cross sectional view of a processing device.
FIG. 2 is a systematic schematic diagram of a fluid control device according to a first embodiment of the present invention.
FIG. 3 is a flowchart of an operating manner of the fluid control device according to the first embodiment of the present invention.
FIG. 4 is a systematic schematic diagram of a fluid control device according to a second embodiment of the present invention.
FIG. 5 is a flowchart of an operating manner of the fluid control device according to the second embodiment of the present invention.
FIG. 6 is a systematic schematic diagram of a fluid control device according to a third embodiment of the present invention.
FIG. 7 is a flowchart of an operating manner of the fluid control device according to the third embodiment of the present invention.
Fig. 8 is a perspective view of a treatment device.
Fig. 9 is a perspective view of a part of the control unit used in a fluid control device according to the invention.
Fig. 10 is a perspective view of a fluid control device according to the invention.

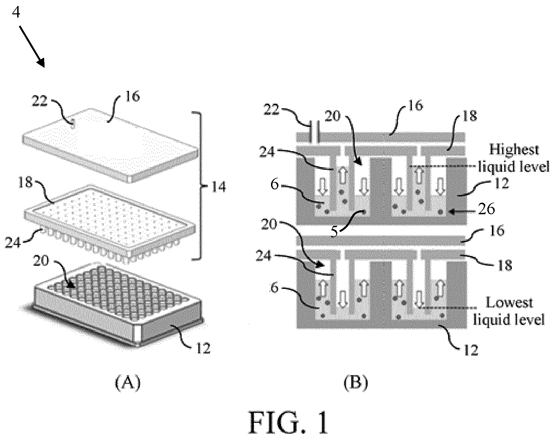

FIG. 1(A) and FIG. 1(B) show a processing device, respectively. The processing device comprises a multiwell plate 12 comprising a plurality of receptacles 20. The embodiment shown in the drawings has 96 receptacles. A fluid sample 26 is located in each of the receptacles 20 wherein the fluid sample 26 comprises a liquid 6 and a plurality of biological particles 5 like cells. The multiwell plate 12 is covered with a lid 14. The lid 14 has a top-layer-of-lid 16 and a bottom-layer-of-lid 18. A space is formed between the top-layer-of-lid 16 and the bottom-layer-of-lid 18 of the lid 14. The top-layer-of-lid 16 is provided with a hollow connection port 22, and the bottom-layer-of-lid 18 is provided with hollow extension tubes 24. Each extension tube 24 of the bottom-layer-of-lid 18 is inserted into the fluid sample 26 in each receptacle 20 of the multiwell plate 12.

An air passage is formed throughout the hollow connection port 22 of the top-layer-of-lid 16, the space between the top-layer-of-lid 16 and the bottom-layer-of-lid 18, and the hollow extension tube 24 of the bottom-layer-of-lid 18. When gas is injected from the connection port 22 of the top-layer-of-lid 16, the gas, through the connection port 22 of the top-layer-of-lid 16, the space between the top-layer-of-lid 16 and the bottom-layer-of-lid 18, and the extension tube 24 of the bottom-layer-of-lid 18, applies pressure to the fluid sample 26 in the extension tube 24, to make the liquid level of the cell suspension in the extension tube 24 fall. When vacuumization is performed through the connection port 22 of the top-layer-of-lid 16, the gas is pumped out through the connection port 22 of the top-layer-of-lid 16, the space between the top-layer-of-lid 16 and the bottom-layer-of-lid 18, and the extension tube 24 of the bottom-layer-of-lid 18 to reduce the pressure applied to the fluid sample 26 in the extension tube 24, so that the liquid level of the fluid sample 26 in the extension tube 24 rises (as shown in FIG. 1(B)).

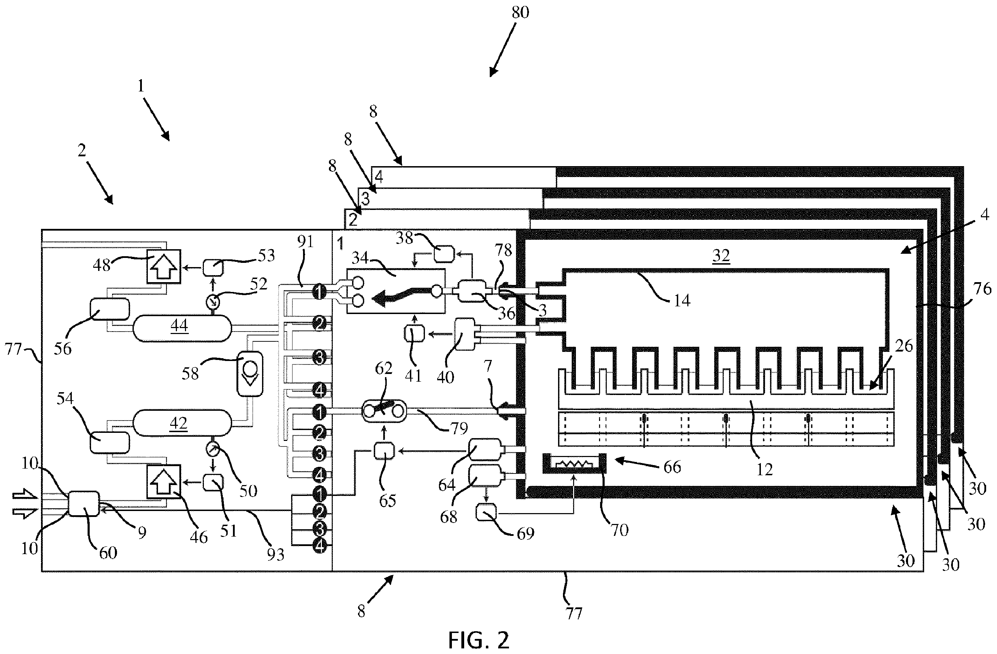

FIG. 2 is a systematic schematic diagram of a fluid control device 80 according to a first embodiment of the present invention. In the first embodiment, a fluid control device 80 includes a control unit 1 and four treatment devices 30. It is clear that the fluid control device 80 can comprise more or less than four treatment devices 30. Each of the treatment devices 30 has the same structure and components so that in the following merely one treatment device 30 is explained. Additionally, the fluid control device 80 comprises the processing device 4 as show in Fig. 1 (A) and (B). The processing device 4 is arranged in a chamber 32 of the treatment device 30. In said case the fluid control device 80 corresponds with a bioreactor. In particular, the fluid control device 80 can comprise four processing device 4 wherein each of the processing devices 4 is arranged in a chamber 32 of the treatment device 30, respectively.

In alternative embodiments a processing device 4 can be used that does not comprise a fluid sample comprising biological particles but only chemical reagents. Alternatively, the processing device 4 can be a microfluidic device, in particular a microfluidic chip.

The control unit 1 comprises one pressure unit 2 and four connection units 8. The connection units 8 have the same structure and components so that in the following merely one connection unit 8 is explained. It is clear that the control unit 1 can comprise more or less than four connection units 8. In particular, the number of connection units 8 can correspond with the number of treatment devices 30.

Additionally, the control unit 1 comprises several subcontrollers, namely a first subcontroller that is named as pressure control 41, a second subcontroller is named as flow controller 38, a third subcontroller is named as mixed gas controller 65, a fourth subcontroller is named as humidity controller 69, a fifth subcontroller is named as pressure controller 51 and a sixth subcontroller is named as vacuum controller 53. The flow controller 38, pressure controller 41, pressure controller 51, vacuum controller 53, mixed gas controller 65, and humidity controller 69 can be integrated into one controller or a plurality of controllers.

The treatment device 30 comprises a chamber 32 surrounded by a housing 76 of the treatment device 30. A humidifier 66 is arranged within the chamber 32. The processing device as shown in Fig. 1 (A) and (B) is also arranged within the chamber 32. The treatment device 30 is mechanically connected with the control unit 1, in particular with a housing 77 of the control unit 1. Additionally, the processing device 4 is fluidically connected with a first control unit outlet 3 and the treatment device 30 is fluidically connected with a second control unit outlet 7. The first control unit outlet 3 is part of a tube 78 of the control unit 1 and the second control unit outlet 7 is part of a further tube 79 of the control unit 1.

The pressure unit 2 comprises a pressurized gas tank 42 and a pump 46. The pump 46 pumps pressurized gas into the gas tank 42. A filter 54 is arranged downstream the pump 46. The filter 54 filters out impurities in the gas pumped by the pump 46 into the pressurized gas tank 42. The pressure unit 2 also comprises pressure controller 51 for controlling the pump 46 and a pressure gauge 50. The pressure gauge 50 gauges pressure of the positive pressure gas in the pressurized tank 42 to generate a positive-pressure gauging signal. The pressure controller 51 receives the positive-pressure gauging signal generated by the pressure gauge 50 and determines whether the positive-pressure gauging signal is less than the critical positive pressure value. If the positive-pressure gauging signal is less than the critical positive pressure value, the pressure controller 51 controls the pump 46 to pump the pressurized gas into the pressurized tank 42; and otherwise, the pump 46 does not pump the gas into the pressurized gas tank 42.

A gas mixer 60 is arranged upstream the pump 46. The gas mixer 60 mixes a plurality of types of gases such as air (atmospheric air in this embodiment) and specific mixed gas (carbon dioxide in this embodiment) that are provided by a gas supply (not shown in the figure) as a mixed gas of mixed-gas preset concentration (that is, preset concentration of carbon dioxide). The mixed gas is pressurized by the pump 46 to be pumped into the pressurized gas tank 42. The gas mixer 60 comprises two inlet 10 for gas inlet and one outlet 9 for discharging the mixed gas.

The pressure unit 2 comprises a vacuum gas tank 44 and a further pump 48. The further pump 48 vacuumizes the vacuum gas tank 44. A vacuum filter 56 is arranged downstream the vacuum gas tank 44 filters out impurities in the gas that is in the vacuum gas tank 44 vacuumized by the further pump 48.

Additionally, the pressure unit 2 comprises a vacuum gauge 52. The vacuum gauge 52 gauges pressure of the negative pressure gas in the vacuum gas tank 44 to generate a negative-pressure gauging signal. The vacuum controller 53 receives the negative-pressure gauging signal generated by the vacuum gauge 52 and determines whether the negative-pressure gauging signal is greater than the critical negative pressure value. If the negative-pressure gauging signal is greater than the critical negative pressure value, the vacuum controller 53 controls the further pump 48 to vacuumize the vacuum gas tank 44; and otherwise, the further pump 48 does not vacuumize the vacuum gas tank 44.

The connection unit 8 comprises a valve 34. The valve 34 is fluidically connected with the tube 78comprising the first control unit outlet 3. Additionally, the valve 34 is fluidically connected with the vacuum gas tank 44 and the pressurized gas tank 42. The pressurized tank 42 provides the positive pressure gas to the valve 34 and the vacuum gas tank 44 provides the negative pressure gas to the valve 34. A check valve 58 of the pressure unit 2 is provided and arranged such that it prevents backstreaming of the positive pressured gas provided by the pressurized gas tank 42 to the valve 34.

The valve 34 alternately delivers positive pressured gas and negative pressured gas to the first control unit outlet 3 and, thus, to the processing device 4. In particular, the positive pressured gas or negative pressured gas is delivered to an inner space of the lid 14 (that is, the space between the top-layer-of-lid 16 and the bottom-layer-of-lid 18). This leads to that positive pressured gas and negative pressured gas can be applied to the fluid sample 26 in each extension tube 24 of the lid 14 in order to form a liquid mixing mechanism of repeatedly pumping up and down the fluid sample 26 in each receptacle 20 of the multiwell plate 12.

A flow sensor 36 of the connection unit 8 can be arranged downstream of the valve 34 and upstream of the first control unit outlet 3. The flow sensor 36 senses gas flows of the positive pressure gas and the negative pressure gas that are delivered by the valve 34 into the processing device 4, in particular the internal space of the lid 14 inside the chamber 32, and generates a flow sensing signal. The flow controller 38 receives the flow sensing signal generated by the flow sensor 36 and determines a difference between the flow sensing signal and a target flow signal, to control an opening size of the valve 34, that is, to control the gas flow of the positive pressure gas and the negative pressure gas that are delivered by the valve 34 into the processing device 4, in particular the internal space of the lid 14, inside the chamber 32.

The connection unit 8 comprises a pressure sensor 40. The pressure sensor 40 senses the pressure in the chamber 32 of the treatment device 30. Additionally, the pressure sensor 20 sense the pressure within the inner space of the lid 14. Based on this sensed pressure signals the pressure sensor 40 generates a pressure sensing signal. The pressure controller 41 of the control unit 1 receives the pressure sensing signal generated by pressure sensor 40 and determines whether the pressure sensing signal is greater than or equal to a critical positive pressure value or less than a critical negative pressure value, to control the valve 34 to be connected to a passage, that is, when the pressure sensing signal of the lid 14 is greater than or equal to the critical positive pressure value, the pressure controller 41 controls the valve 34 to be connected to a passage of the negative pressure gas (a passage connecting the vacuum gas tank 44); and when the pressure sensing signal is less than or equal to the critical negative pressure value, the pressure controller 41 controls the valve 34 to be connected to a passage of the positive pressure gas (a passage connecting the pressurized gas tank 42).

The connection unit 8 comprises additionally a further valve 62 that is fluidically connected to the pressurized gas tank 42 to deliver the mixed gas in the pressurized tank 42 into the chamber 32 of the treatment device 30. The valve 62 is not fluidically connected to the vacuum gas tank 44. The connection unit 8 comprises a mixed gas sensor 64 that senses the concentration of the mixed gas in the chamber 32 and generates a mixed-gas sensing signal.

The mixed gas controller 65 receives the mixed-gas sensing signal generated by the mixed gas sensor 64 and determines whether the mixed-gas sensing signal is greater than or equal to a mixed-gas preset concentration value, to control the further valve 62 to be switched on or off. Additionally, the mixed gas controller 65 controls the gas mixer 60 to mix the air and the specific mixed gas as the mixed gas of the mixed-gas preset concentration. This means, when the mixed-gas sensing signal is greater than or equal to the mixed-gas preset concentration value, the mixed gas controller 65 controls the further valve 62 to be switched off, and when the mixed-gas sensing signal is less than the mixed-gas preset concentration value, the mixed gas controller 65 controls the further valve 62 to be connected to the pressurized tank 42 and controls the gas mixer 60 to mix the plurality of types of gases provided by the gas supply as the mixed gas of the mixed-gas preset concentration.

The humidifier 66 is provided with a heater 70. The heater 70 heats water in the humidifier 66 to increase humidity in the chamber 32. A humidity sensor 68 of the connection unit 8 senses the humidity in the chamber 32 to generate a humidity sensing signal.

The humidity controller 69 receives the humidity sensing signal generated by the humidity sensor 68 and determines whether the humidity sensing signal is less than a preset critical humidity value, to control the heater 70 of the humidifier 66 to perform heating. This means, when the humidity sensing signal is less than the preset critical humidity value, the humidity controller 69 controls the heater 70 of the humidifier 66 to heat the water in the humidifier 66 to increase the humidity in the chamber 32; and when the humidity sensing signal is greater than or equal to the preset critical humidity value, the humidity controller 69 does not control the heater 70 of the humidifier 66 to perform heating.

The control unit 1 comprises the pressure unit 2 and the connection 8. The control unit 1 is adapted to process the liquid sample in the processing device 4 by applying positive or negative pressure to the first control unit outlet 3 by means of the connection unit 8 that is fluidically connected with the pressure unit 2. In particular, the valve 32 is fluidically connected with the pressure unit 2. Additionally, the control unit 1 is adapted to control a physical state in the chamber 32 of the treatment device 30. Thereto, the further valve 65 and/or the humidifier 66 are controlled. In particular, the control unit 1 is adapted to control the valve 65 such that the pressure unit 2 is fluidically connected or disconnected with a second control unit outlet 7.

As can be seen in fig. 2 the control unit 1 comprises a plurality of tubes 91. Said tubes 91 fluidically connect the components of the pressure unit 2 with the components of the connection unit 8. The fluid control device 80 shown in fig. 2 comprises four treatment devices 30. Therefore, the tubes 91 have four branches leading to the respective connection unit 8. The tube branches are numbered wherein the number is placed in a black circle.

The gas mixer 60 is connected with the gas mixed controller 65 of the respective connection unit 8 by means of electric lines 93. The electric line also branches wherein the respective branch is numbered and placed in a black circle.

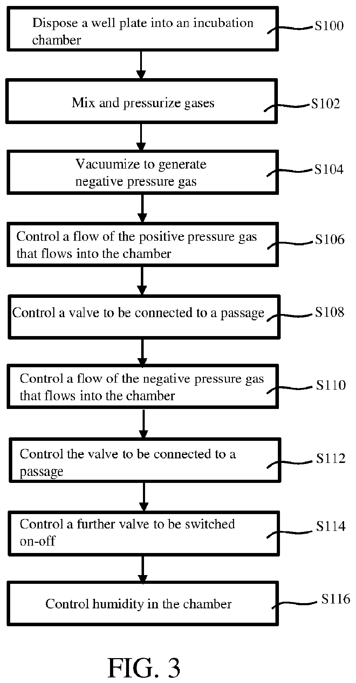

FIG. 3 is a flowchart of an operating manner of the fluid control device 80 according to the first embodiment of the present invention. The flow of steps in FIG. 3 is described with reference to the apparatus and system in FIG. 1(A) and FIG. 1(B) and FIG. 2.

In the first embodiment, the fluid sample 26 is injected into each receptacle 20 of the multiwell plate 12. The multiwell plate 12 is covered with the lid 14 to make each extension tube 24 of the lid 14 inserted into the fluid 26 in the each receptacle 20 of the multiwell 12. The multiwell plate 12 and the lid 14 are disposed in the chamber 32 of the treatment device (step S100).

The gas mixer 60 mixes a plurality of types of gases such as air (atmospheric air in this embodiment) and specific mixed gas (carbon dioxide in this embodiment) that are provided by a gas supply as a mixed gas of mixed-gas preset concentration (that is, preset concentration of carbon dioxide in this embodiment). The pump 46 pressurize the mixed gas mixed by the gas mixer 60 as positive pressure gas to be pumped into the pressurized tank 42. The pressurized tank 42 that is connected to the valve 34 provides the positive pressure gas to the valve 34 (step S102).

The filter 54 filters out impurities in the mixed gas pumped by the pump 46 into the pressurized tank 42. The check valve 58 prevents backstreaming of the positive pressure gas provided by the pressurized tank 42 to the valve 34. The pressure gauge 50 gauges pressure of the positive pressure gas in the pressurized tank 42 to generate a positive-pressure gauging signal. The pressure controller 51 receives the positive-pressure gauging signal generated by the pressure gauge 50 and determines whether the positive-pressure gauging signal is less than the critical positive pressure value. If the positive-pressure gauging signal is less than the critical positive pressure value, the pressure controller 51 controls the pressure pump 46 to pump the pressurized positive pressure gas into the pressurized tank 42; and otherwise, the pump 46 does not pump the gas into the pressurized tank 42.

The pump 48 vacuumizes the vacuum gas tank 44 to make the gas in the vacuum gas tank 44 to become negative pressure gas. The vacuum gas tank 44 that is connected to the valve 34 of provides the negative pressure gas to the valve 34 (step S104).

The vacuum filter 56 filters out impurities in the negative pressure gas that is in the vacuum gas tank 44 vacuumized by the vacuum pump 48. The vacuum gauge 52 gauges pressure of the negative pressure gas in the vacuum gas tank 44 to generate a negative-pressure gauging signal. The vacuum controller 53 receives the negative-pressure gauging signal generated by the vacuum gauge 52 and determines whether the negative-pressure gauging signal is greater than the critical negative pressure value. If the negative-pressure gauging signal is greater than the critical negative pressure value, the vacuum controller 53 controls the vacuum pump 48 to vacuumize the vacuum gas tank 44; and otherwise, the vacuum pump 48 does not vacuumize the vacuum gas tank 44.

The valve 34 is connected to a passage comprising the tubes 91 and connecting the pressurized tank 42 in which there is the positive pressure gas, and the valve 34 delivers the positive gas in the pressurized tank 42 via the first control unit outlet 3 into the processing device 4, in particular the internal space of the lid 14, inside the chamber 32 to make the positive pressure gas apply positive pressure to the fluid sample 26 in each extension tube 24 of the lid 14.

The flow sensor 36 senses a gas flow of the positive pressure gas that is delivered by the valve 34 via the first control unit outlet 3 into the processing device 4 and generates a flow sensing signal. The flow controller 38 receives the flow sensing signal generated by the flow sensor 36 and determines a difference between the flow sensing signal and a target flow signal, and the flow controller 38 controls an opening size of the valve 34 according to the difference (step S106).

The pressure sensor 40 senses the positive pressure of the positive pressure gas in the chamber 32 and the internal space of the lid 14 inside the chamber 32 and generates a positive-pressure sensing signal. The pressure controller 41 receives the positive-pressure sensing signal generated by pressure sensor 40 and determines whether the positive-pressure sensing signal is greater than or equal to a critical positive pressure value. If the positive-pressure sensing signal is greater than or equal to the critical positive pressure value, the pressure controller 41 controls the valve 34 to be connected to a passage comprising the tubes 91 and connecting the vacuum gas tank 44 in which there is the negative pressure gas (step S108).

The valve 34 is connected to a passage connecting the vacuum gas tank 44 in which there is the negative pressure gas, and the valve 34 delivers the negative pressure gas in the vacuum gas tank 44 into the internal space of the lid 14 inside the chamber 32 apply negative pressure to the fluid sample 26 in each extension tube 24 of the lid 14.

The flow sensor 36 senses a gas flow of the negative pressure gas that is delivered by the valve 34 into the internal space of the lid 14 inside the incubation chamber 32 and generates a flow sensing signal. The flow controller 38 receives the flow sensing signal generated by the flow sensor 36 and determines a difference between the flow sensing signal and the target flow signal, and the flow controller 38 controls an opening size of the valve 34 according to the difference (step S110).

The pressure sensor 40 senses the negative pressure of the negative pressure gas in the chamber 32 and the internal space of the lid 14 inside the chamber 32 and generates a negative-pressure sensing signal.

The pressure controller 41 receives the negative-pressure sensing signal generated by pressure sensor 40 and determines whether the negative-pressure sensing signal is less than or equal to a critical negative pressure value. If the negative-pressure sensing signal is less than or equal to the critical negative pressure value, the pressure controller 41 controls the valve 34 to be connected to a passage connecting the pressurized tank 42 in which there is the positive pressure gas (step S112).

Steps S106, S108, S110, and S112 are repeated to make the positive pressure gas and the negative pressure pressure gas that flow into the chamber 32 through the valve 34 and the first control unit outlet 3 to apply the positive pressure and the negative pressure to the fluid sample 26 in each extension tube 24 of the lid 14, to form a liquid mixing mechanism of repeatedly pumping up and down the fluid sample 26 in in each receptacle 20 of the multiwell plate 12.

The further valve 62 that is connected to the pressurized tank 42 delivers the mixed gas in the pressurized tank 42 via the second control unit outlet into the chamber 32. The mixed gas sensor 64 senses the concentration of the mixed gas in the incubation chamber 32 and generates a mixed-gas sensing signal.

The mixed gas controller 65 receives the mixed-gas sensing signal generated by the mixed gas sensor 64 and determines whether the mixed-gas sensing signal is greater than or equal to a mixed-gas preset concentration value, to control the further valve 62 to be switched on or off and control the gas mixer 60 to mix the air and the specific mixed gas as the mixed gas of the mixed-gas preset concentration. That means, when the mixed-gas sensing signal is greater than or equal to the mixed-gas preset concentration value, the mixed gas controller 65 controls the further valve 62 to be switched off, and when the mixed-gas sensing signal is less than the mixed-gas preset concentration value, the mixed gas controller 65 controls the valve 62 to be connected to the pressurized tank 42 and controls the gas mixer 60 to mix the plurality of types of gases provided by the gas supply as the mixed gas of the mixed-gas preset concentration (step S114).

The humidifier 66 is disposed in the chamber 32. The humidifier 66 is provided with a heater 70. The heater 70 heats water in the humidifier 66 to increase humidity in the chamber 32. The humidity sensor 68 senses the humidity in the incubation chamber 32 to generate a humidity sensing signal.

The humidity controller 69 receives the humidity sensing signal generated by the humidity sensor 68 and determines whether the humidity sensing signal is less than a preset critical humidity value, to control the heater 70 of the humidifier 66 to perform heating, that is, if the humidity sensing signal is less than the preset critical humidity value, the humidity controller 69 controls the heater 70 of the humidifier 66 to heat the water in the humidifier 66 to increase the humidity in the incubation chamber 32; and if the humidity sensing signal is greater than or equal to the preset critical humidity value, the humidity controller 69 does not control the heater 70 of the humidifier 66 to perform heating (step S116).

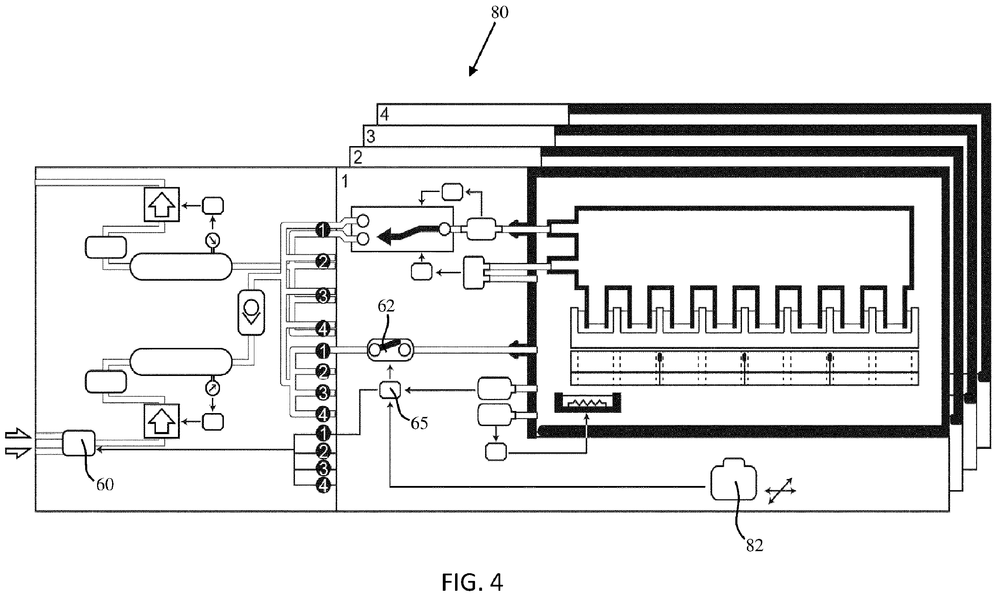

FIG. 4 is a systematic schematic diagram of a fluid control device 80 according to a second embodiment of the present invention. The difference between a system structure of a fluid control device 80 in the second embodiment and a system structure of the fluid control device 80 in the first embodiment lays is that the fluid control device 80 in the second embodiment is provided with an acquisition unit like an optical imaging apparatus 82 which is disposed on a movable electric platform to perform scanning, or is provided with four optical imaging apparatus 82. The remaining apparatuses in the fluid control device 80 in the second embodiment are the same as those in the fluid control device 80 in the first embodiment and use the same element symbols. To simplify the description, one optical imaging apparatus 82 in each incubation chamber 32 is used for the description below. The same operations and functions are applicable to the remaining apparatuses.

The optical imaging apparatus 82 tests a liquid pH value and liquid dissolved oxygen content of fluid sample 26 in the receptacle 20 of the multiwell plate 12 of the incubation chamber 32 in an optical testing manner. The mixed gas controller 65 receives the liquid pH value and the liquid dissolved oxygen content tested by the optical imaging apparatus 82 and determines whether the liquid pH value is greater than a critical liquid pH value and whether the liquid dissolved oxygen content is less than critical liquid dissolved oxygen content, to control the further valve 62 to be switched on or off and control a gas mixer 60 to mix a plurality of types of gases provided by a gas supply as a mixed gas of the preset liquid pH value and the preset liquid dissolved oxygen content, that is, when the tested liquid pH value is less than or equal to the critical liquid pH value and the liquid dissolved oxygen content is greater than or equal to the critical liquid dissolved oxygen content, the mixed gas controller 65 controls the further valve 62 to be switched off; and when the tested liquid pH value is greater than the critical liquid pH value and the liquid dissolved oxygen content is less than the critical liquid dissolved oxygen content, the mixed gas controller 65 controls the valve 62 to be connected to the pressurized gas tank 42 and controls the gas mixer 60 to mix the plurality of types of gases provided by the gas supply as the mixed gas of the preset liquid pH value and the preset liquid dissolved oxygen content.

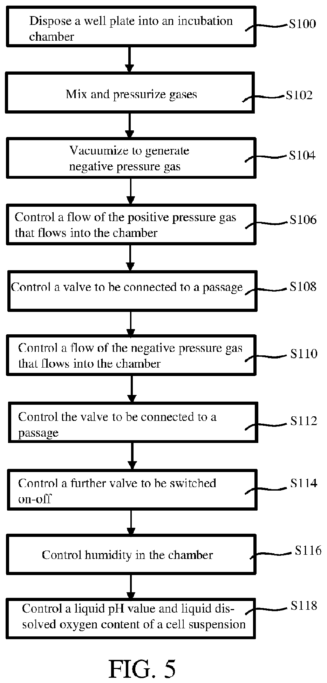

FIG. 5 is a flowchart of an operating manner of incubating cells or microbes in the fluid control device according to the second embodiment of the present invention. The flow of steps in FIG. 5 is described with reference to the apparatus and system in FIG. 1(A) and FIG. 1(B) and FIG. 4.

Functional operations of flow steps S100, S102, S104, S106, S108, S110, S112, S114, and S116 in the second embodiment are the same as those of flow steps S100, S102, S104, S106, S108, S110, S112, S114, and S116 in the first embodiment, and therefore descriptions are omitted herein. The difference lies in that a functional operation of step S118 of controlling the liquid pH value and the liquid dissolved oxygen content of the cell suspension is added in the second embodiment.

The optical imaging apparatus 82 tests a liquid pH value and liquid dissolved oxygen content of fluid sample 26 in the receptacle 20 of the multiwell plate 12 of the chamber 32 in an optical testing manner. The mixed gas controller 65 receives the liquid pH value and the liquid dissolved oxygen content tested by the optical imaging apparatus 82 and determines whether the liquid pH value is greater than a critical liquid pH value and whether the liquid dissolved oxygen content is less than critical liquid dissolved oxygen content, to control the further valve 62 to be switched on or off and control the gas mixer 60 to mix a plurality of types of gases provided by a gas supply as a mixed gas of the preset liquid pH value and the preset liquid dissolved oxygen content, that is, when the tested liquid pH value is less than or equal to the critical liquid pH value and the liquid dissolved oxygen content is greater than or equal to the critical liquid dissolved oxygen content, the mixed gas controller 65 controls the further valve 62 to be switched off; and when the tested liquid pH value is greater than the critical liquid pH value and the liquid dissolved oxygen content is less than the critical liquid dissolved oxygen content, the mixed gas controller 65 controls the further valve 62 to be connected to a pressurized gas tank 42 and controls the gas mixer 60 to mix the plurality of types of gases provided by the gas supply as the mixed gas of the preset liquid pH value and the preset liquid dissolved oxygen content (step S118).

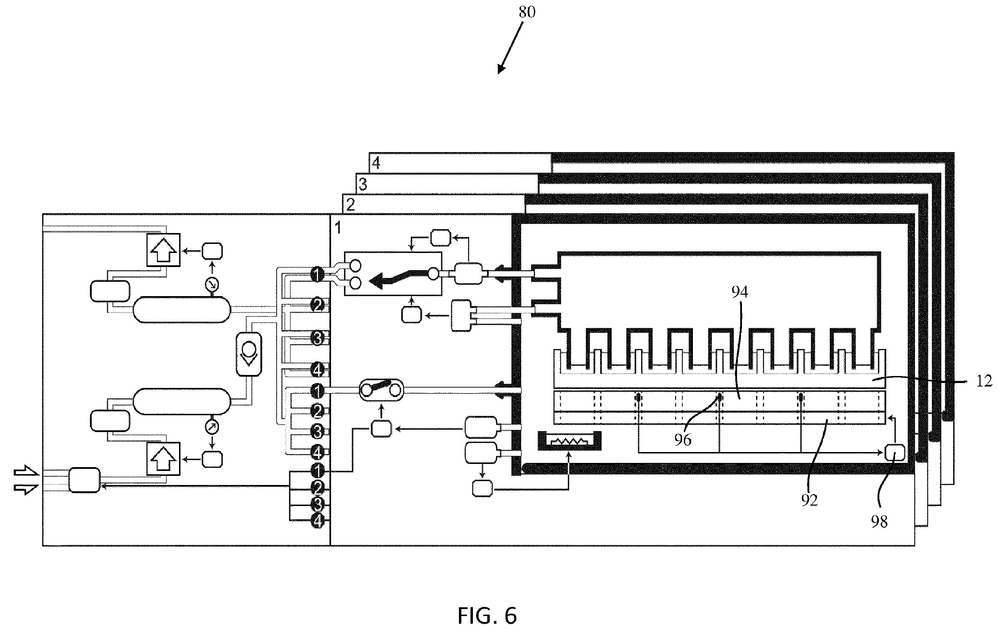

FIG. 6 is a systematic schematic diagram of a fluid control device 80 according to a third embodiment of the present invention. The difference between a system structure of a fluid control device 80 in the third embodiment and the system structure of the fluid control device 30 in the first embodiment lies in that the fluid control device 80 in the third embodiment is provided with four additional heating units. Each of the heating units comprises a heating plate 92, a thermal block 94, a plurality of temperature sensors 96, and a temperature controller 98. The remaining apparatuses in the fluid control device 80 in the third embodiment are the same as those in the fluid control device 80 in the first embodiment and use the same element symbols. To simplify the description, one heating plate 92, one thermal block 94, and one temperature controller 98 in each incubation chamber 32 are used for the description below. The same operations and functions are applicable to the remaining apparatuses.

The thermal block 94 is disposed in the chamber 32. A multiwell plate 12 is disposed on the thermal block 94. The heating plate 92 is disposed in the chamber 32. The thermal block 94 is disposed on the heating plate 92. The heating plate 92 heats the thermal block 94 so that the thermal block 94 indirectly heats a multiwell plate 12.

The plurality of temperature sensors 96 between the thermal block 94 and the multiwell plate 12 sense a plurality of heating temperature values of heating performed on the multiwell plate 12 by the thermal block 94. The temperature controller 98 receives the plurality of heating temperature values generated by the plurality of temperature sensors 96 and determines whether the plurality of heating temperature values are less than a preset critical temperature value, to control the heating plate 92 to heat the thermal block 94, that is, if the plurality of heating temperature values are less than the preset critical temperature value, the temperature controller 98 controls the heating plate 92 to heat the thermal block 94 so that the thermal block 94 indirectly heats the multiwell plate 12; and if all the plurality of temperature sensing signals are greater than or equal to the preset critical temperature value, the temperature controller 98 does not control the heating plate 92 to heat the thermal block 94.

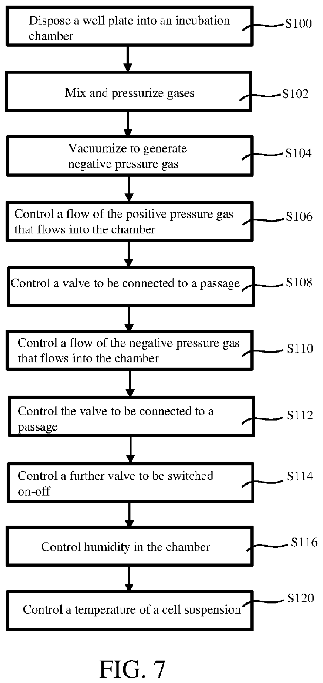

FIG. 7 is a flowchart of an operating manner of incubating cells in the fluid control device according to the third embodiment of the present invention. The flow of steps in FIG. 7 is described with reference to the apparatus and system in FIG. 1(A) and FIG. 1(B) and FIG. 6.

Functional operations of flow steps S100, S102, S104, S106, S108, S110, S112, S114, and S116 in the third embodiment are the same as those of flow steps S100, S102, S104, S106, S108, S110, S112, S114, and S116 in the first embodiment, and therefore descriptions are omitted herein. The difference lies in that a functional operation of step S120 of controlling a liquid temperature of the cell suspension is added in the third embodiment.

A multiwell plate 12 is disposed on the thermal block 94 disposed in the chamber 32. The thermal block 94 is disposed on the heating plate 92 disposed in the chamber 32. The heating plate 92 heats the thermal block 94 so that the thermal block 94 indirectly heats the multiwell plate 12.

The plurality of temperature sensors 96 between the thermal block 94 and the multiwell plate 12 sense a plurality of heating temperature values of heating performed on the multiwell plate 12 by the thermal block 94. The temperature controller 98 receives the plurality of heating temperature values generated by the plurality of temperature sensors 96 and determines whether the plurality of heating temperature values are less than a preset critical temperature value, to control the heating plate 92 to heat the thermal block 94, that is, if the plurality of heating temperature values are less than the preset critical temperature value, the temperature controller 98 controls the heating plate 92 to heat the thermal block 94 so that the thermal block 94 indirectly heats the multiwell plate 12; and if all the plurality of temperature sensing signals are greater than or equal to the preset critical temperature value, the temperature controller 98 does not control the heating plate 92 to heat the thermal block 94 (step S120).

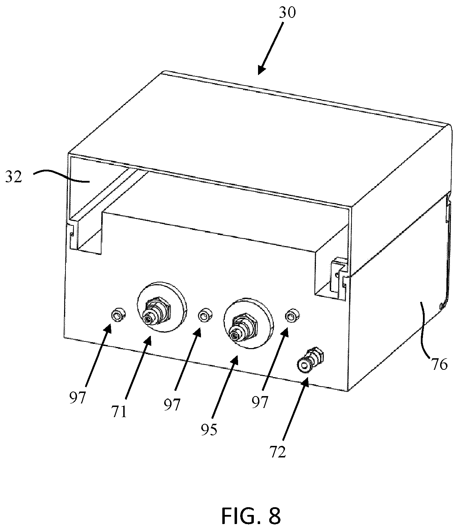

Fig. 8 shows a perspective view of the treatment device 30. In particular, the side of the treatment device 30 is shown what comes into contact with the control unit 1. The treatment device 30 can be used in each of the aforementioned fluid control devices 80 and comprises a fluid connector 71 for fluidically connecting the tube 78 of the control unit 1 with the processing device 4. The fluidic connector 71 protrudes from the side of the treatment device 30 and comprises a hollow portion. The tube 78 of the control unit 1 is inserted into the hollow portion of the fluid connector 71. Additionally, the treatment device 30 comprises a further fluid connector 72. The further fluid connector 72 is used to fluidically connect the further tube 79 of the control unit 1 with the chamber 32 of the treatment device 30. The treatment device 30 also comprises an additional fluid connector 95. The additional fluid connector 95 is used to connect the pressure sensor 40 with the processing device 4 and the chamber 32. The further and additional fluid connector 72, 95 protrude from the same of the treatment device 30 as the fluid connector 71.

The treatment device 30 comprises connection areas 97 used for mechanically connecting the treatment device 30 with the control unit 1. In particular, the connection areas 97 can have through hole for receiving a connection means like a screw with which the two components are mechanically connected.

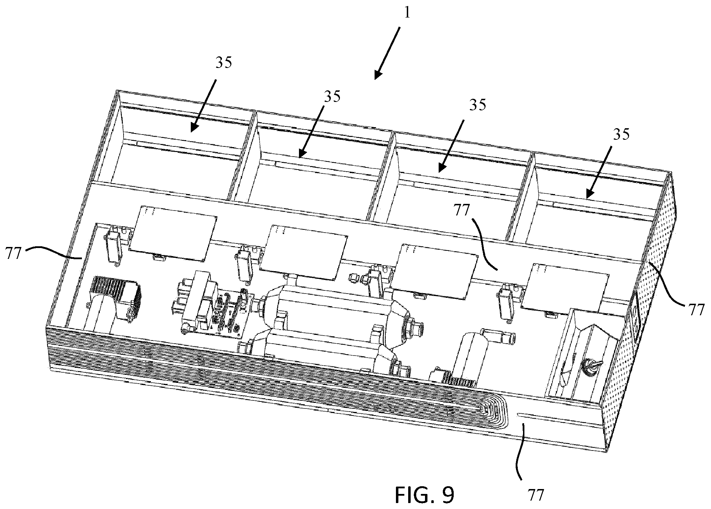

Fig. 9 is a perspective view of a part of the control unit 1. Fig. 9 does not show all aforementioned components of the control unit 1. In particular, fig. 9 shows not an upper part covering the control unit 1, the tubes and the fluidic connection between the control unit 1 and the treatment device 30 and the processing device 4 is not shown. The aforementioned fluid control devices 80 can comprise a control unit 1 as shown in fig. 9.

Fig. 9 shows that the control unit 1 has a housing 77 surrounding an inner space in which the pressure unit 2 and the connection unit 8 are arranged. The housing 77 is in direct contact with the housing 76 of the treatment device 30 when the treatment device 30 and the control unit 1 are fluidically and mechanically connected. The control 1 comprises receiving portions 35 for receiving a treatment module 30, respectively. In particular, the control unit 1 comprises four receiving portions 35 for receiving one treatment module 30.

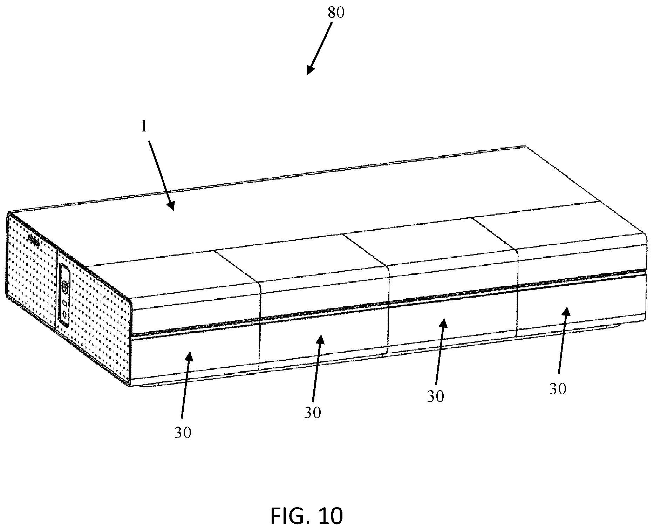

Fig. 10 shows a perspective view of the fluid control device 80. The fluid control device 80 can be one of the fluid control devices as described above. The fluid control device 80 comprises the control unit 1, in particular a control unit 1 as shown in fig. 9, and a plurality of treatment devices 30 that are fluidically and mechanically connected with the control unit 1. The treatment devices 30 are arranged adjacent to each other along a length axis of the fluid control device 80. Additionally, each of the treatment devices 30 is arranged in one receiving portion 35 of the control unit 1.

### Reference Signs

- 1: Control unit
- 2: Pressure unit
- 3: First control unit outlet
- 4: Processing device
- 5: Biological particle
- 6: Liquid
- 7: Second control unit outlet
- 8: Connection unit
- 9: Outlet
- 10: Inlet
- 12: Multiwell plate
- 14: Lid
- 16: Top-layer-of-lid
- 18: Bottom-layer-of-lid
- 20: Receptacle
- 22: Connection port
- 24: Extension tube
- 26: Fluid sample
- 30: Treatment device
- 32: Chamber
- 34: Valve
- 35: Receiving portion
- 36: Flow sensor
- 38: Flow controller
- 40: Pressure sensor
- 41: Pressure controller
- 42: Pressurized gas tank
- 44: Vacuum gas tank
- 46: Pump
- 48: Further pump
- 50: Pressure gauge
- 51: Pressure controller
- 52: Vacuum gauge
- 53: Vacuum controller
- 54: Filter
- 56: Further gas filter
- 58: Check valve
- 60: Gas mixer
- 62: Further valve
- 64: Mixed gas sensor
- 65: Mixed gas controller
- 66: Humidifier
- 68: Humidity sensor
- 69: Humidity controller
- 70: Heater
- 71: Fluid connector
- 72: Further fluid connector
- 76: Housing of treatment device
- 77: Housing of control unit
- 78: Tube
- 79: Further tube
- 80: Fluid control device
- 82: Optical imaging apparatus
- 91: Tube
- 92: Heating plate
- 93: Electric line
- 94: Thermal block
- 95: Additional fluid connector
- 96: Temperature sensor
- 97: Connection area
- 98: Temperature controller

## Claims

1. Fluid control device (80) comprising:
at least one treatment device (30) having a chamber (32) for receiving a processing device (4),
at least one processing device (4) which is arranged in the chamber (32) of the treatment device (30), and a control unit (1) comprising
a pressure unit (2) for providing positive and/or negative pressure,
at least one first control unit outlet (3) being fluidically connected to the processing device (4) comprising at least one receptacle (20) for receiving a fluid sample (26),
at least one second control unit outlet (7) being fluidically connected to the treatment device (30), and
a connection unit (8) by means of which the pressure unit (2) is fluidically connected with the first control unit outlet (3) and/or with the second control unit outlet (7) wherein
the control unit (1) is adapted to control the processing of the fluid sample (26) in the processing device (4) by applying a positive or negative pressure provided by the pressure unit (2) to the first control unit outlet (3) by means of the connection unit (8) and to control a physical state in the chamber (32) of the treatment device (4).

2. Fluid control device (80) according to claim 1, **characterized in that** the control unit (1) is adapted to control the physical state in the chamber (32) of the treatment device by fluidically connecting or disconnecting the pressure unit (2) with the second control unit outlet (7) by means of the connection unit (8).

3. Fluid control device (80) according to claim 1 or 2, **characterized in that** the pressure unit (2) comprises a pressurized gas tank (42) having a positive pressure wherein the pressurized gas tank (42) is fluidically connectable or connected to the first control unit outlet (3) and/or the second control unit outlet (7).

4. Fluid control device (80) according to one of the claims 1 to 3, **characterized in that** the pressure unit comprises a vacuum gas tank (44) having a negative pressure wherein the vacuum gas tank (44) is fluidically connectable or connected to the first control unit outlet (3) and/or the second control unit outlet (7).

5. Fluid control device (80) according to one of the claims 1 to 4, **characterized in that**
a. the control unit (2) is adapted to supply the same gas to the treatment device (30) and to the processing device (4) and/or **in that**
b. the pressurized gas tank (42) supplies the same gas to the first control unit outlet (3) and the second control unit outlet (7).

6. Fluid control device (80) according to one of the claims 3 to 5, **characterized in that** the connection unit comprises at least one valve (34) for selectively fluidically connecting the pressurized gas tank (42) or the vacuum gas tank (44) with the first control unit outlet (3).

7. Fluid control device (80) according to one of the claims 3 to 6, **characterized in that** the connection unit (8) comprises at least one further valve (62) that in a first position fluidically connects the pressurized gas tank (42) with the treatment device (30) and in a second position separates the fluidically connection between the pressurized gas tank (42) and the treatment device (30).

8. Fluid control device (80) according to claim 6 or 7, **characterized in that** the control unit (1) comprises at least one controller for controlling the valve (34) and/or the further valve (62).

9. Fluid control device (80)according to claim 8, **characterized in that**
a. the controller controls the valve (34) based on a gas flow flowing through the first control unit outlet (3) and/or **in that**
b. the controller controls the valve (34) based on a physical state, in particular pressure, in the treatment device (36) and on a physical state, in particular a pressure, of the processing device (4).

10. Fluid control device (80) according to one of the claims 1 to 9, **characterized in that** the control unit comprises
a. a plurality of first control unit outlets (3) wherein each of the plurality of first control unit outlets (3) is fluidically connectable to one processing device (4) and/or
b. a plurality of second control unit outlets (7) wherein each of the plurality of second control unit outlets (7) is fluidically connectable to one treatment device (30).

11. Fluid control device (80) according to claim 10, **characterized in that**
a. the control unit (1) comprises merely one pressure unit (2) and/or **in that**
b. the control unit (1) comprises several connection units (8) and/or **in that**
c. the pressurized gas tank (42) is connectable or connected with each of the plurality of first control unit outlets (3) and/or with each of the plurality of second control unit outlets (7) and/or **in that**
d. the vacuum gas tank (44) is connectable or connected with each of the plurality of first control unit outlets (3).

12. Fluid control device (80) according to claim 10 or 11, **characterized in that** the control unit comprises
a. a plurality of valves (32) wherein each of the valves (32) is fluidically connected with one of the plurality of first control unit outlets (3) and with the pressurized gas tank (42) and the vacuum gas tank (44) and/or
b. a plurality of valves (32) wherein each of the valves (32) is fluidically connected with one of the plurality of second control unit outlets (7) and with the pressurized gas tank (42).

13. Fluid control device (80) according to one of the claims 1 to 12, **characterized in that**
a. the processing device (4) comprises at least one receptacle (20) for receiving the fluid sample (26) and a lid (14) covering the receptacle (20) or **in that**
b. the processing device (4) comprises at least one receptacle (20) for receiving the fluid sample (26) and a lid (14) covering the receptacle (20) wherein the lid (14) comprises at least one extension tube (24) extending into the receptacle (20) and being fluidically connected to the first control unit outlet (3).

14. Fluid control device (80) according to one of the claims 1 to 13, **characterized in that** the controller controls the valve (32) such that a positive pressure provided by the pressurized gas tank (42) is applied via the lid (14) in the receptacle (20) or a negative pressure provided by the vacuum gas tank (44) is applied via the lid (14) in the receptacle (20).

15. Fluid control device (80) according to one of the claims 1 to 14, **characterized in that** the controller controls the further valve (62) such that a predetermined physical state is achieved in the chamber (32) of the treatment device (30).

## Patentansprüche

1. Fluidsteuerungsvorrichtung (80), umfassend:
mindestens eine Behandlungsvorrichtung (30), die eine Kammer (32) zur Aufnahme einer Verarbeitungsvorrichtung (4) aufweist,
mindestens eine Verarbeitungsvorrichtung (4), die in der Kammer (32) der Behandlungsvorrichtung (30) angeordnet ist, und eine Steuereinheit (1), umfassend
eine Druckeinheit (2) zum Bereitstellen von Über- und/oder Unterdruck,
mindestens einen ersten Steuereinheitsausgang (3), der mit der Verarbeitungsvorrichtung (4) fluidisch verbunden ist, die mindestens einen Behälter (20) zur Aufnahme einer Fluidprobe (26) umfasst,
mindestens einen zweiten Steuereinheitsausgang (7), der mit der Behandlungsvorrichtung (30) fluidisch verbunden ist, und
eine Verbindungseinheit (8), mittels der die Druckeinheit (2) mit dem ersten Steuereinheitsausgang (3) und/oder mit dem zweiten Steuereinheitsausgang (7) fluidisch verbunden ist, wobei
die Steuereinheit (1) dazu geeignet ist, die Verarbeitung der Fluidprobe (26) in der Verarbeitungsvorrichtung (4) durch Anwenden eines von der Druckeinheit (2) bereitgestellten Über- oder Unterdrucks auf den ersten Steuereinheitsausgang (3) mittels der Verbindungseinheit (8) zu steuern und einen physikalischen Zustand in der Kammer (32) der Behandlungsvorrichtung (4) zu steuern.

2. Fluidsteuerungsvorrichtung (80) nach Anspruch 1, **dadurch gekennzeichnet, dass** die Steuereinheit (1) dazu geeignet ist, den physikalischen Zustand in der Kammer (32) der Behandlungsvorrichtung zu steuern, indem sie die Druckeinheit (2) mittels der Verbindungseinheit (8) mit dem Auslass (7) der zweiten Steuereinheit fluidisch verbindet oder trennt.

3. Fluidsteuerungsvorrichtung (80) nach Anspruch 1 oder 2, **dadurch gekennzeichnet, dass** die Druckeinheit (2) einen Druckgasspeicher (42) umfasst, der einen Überdruck aufweist, wobei der Druckgasspeicher (42) mit dem ersten Steuereinheitsausgang (3) und/oder dem zweiten Steuereinheitsausgang (7) fluidisch verbindbar oder verbunden ist.

4. Fluidsteuerungsvorrichtung (80) nach einem der Ansprüche 1 bis 3, **dadurch gekennzeichnet, dass** die Druckeinheit einen Vakuumgasspeicher (44) umfasst, der einen Unterdruck aufweist, wobei der Unterdruckgasbehälter (44) mit dem ersten Steuereinheitsauslass (3) und/oder dem zweiten Steuereinheitsauslass (7) fluidisch verbindbar oder verbunden ist.

5. Fluidsteuerungsvorrichtung (80) nach einem der Ansprüche 1 bis 4, **dadurch gekennzeichnet, dass**
a. die Steuereinheit (2) dazu geeignet ist, dasselbe Gas der Behandlungsvorrichtung (30) und der Verarbeitungsvorrichtung (4) zuzuführen, und/oder dass
b. der Druckgasspeicher (42) dasselbe Gas dem ersten Steuereinheitsausgang (3) und dem zweiten Steuereinheitsausgang (7) zuführt.

6. Fluidsteuerungsvorrichtung (80) nach einem der Ansprüche 3 bis 5, **dadurch gekennzeichnet, dass** die Verbindungseinheit mindestens ein Ventil (34) umfasst, um selektiv den Druckgasspeicher (42) oder den Vakuumgasspeicher (44) mit dem ersten Steuereinheitsausgang (3) fluidisch zu verbinden.

7. Fluidsteuerungsvorrichtung (80) nach einem der Ansprüche 3 bis 6, **dadurch gekennzeichnet, dass** die Verbindungseinheit (8) mindestens ein weiteres Ventil (62) umfasst, das in einer ersten Position den Druckgasspeicher (42) mit der Behandlungsvorrichtung (30) fluidisch verbindet und in einer zweiten Position die Fluidverbindung zwischen dem Druckgasspeicher (42) und der Behandlungsvorrichtung (30) trennt.

8. Fluidsteuerungsvorrichtung (80) nach Anspruch 6 oder 7, **dadurch gekennzeichnet, dass** die Steuereinheit (1) mindestens eine Steuerung zum Steuern des Ventils (34) und/oder des weiteren Ventils (62) umfasst.

9. Fluidsteuerungsvorrichtung (80) nach Anspruch 8, **dadurch gekennzeichnet, dass**
a. die Steuerung das Ventil (34) basierend auf einem durch den ersten Steuereinheitsausgang (3) strömenden Gasfluss steuert und/oder dass
b. die Steuerung das Ventil (34) basierend auf einem physikalischen Zustand, insbesondere einem Druck, in der Behandlungsvorrichtung (36) und auf einem physikalischen Zustand, insbesondere einem Druck, der Verarbeitungsvorrichtung (4) steuert.

10. Fluidsteuerungsvorrichtung (80) nach einem der Ansprüche 1 bis 9, **dadurch gekennzeichnet, dass** die Steuereinheit
a. eine Vielzahl von ersten Steuereinheitsauslässen (3), wobei jeder der Vielzahl von ersten Steuereinheitsauslässen (3) fluidisch mit einer Verarbeitungsvorrichtung (4) verbindbar ist und/oder
b. eine Vielzahl von zweiten Steuereinheitsauslässen (7) umfasst, wobei jeder der Vielzahl von zweiten Steuereinheitsauslässen (7) fluidisch mit einer Behandlungsvorrichtung (30) verbindbar ist.

11. Fluidsteuerungsvorrichtung (80) nach Anspruch 10, **dadurch gekennzeichnet, dass**
a. die Steuereinheit (1) lediglich eine Druckeinheit (2) umfasst und/oder dass
b. die Steuereinheit (1) mehrere Verbindungseinheiten (8) umfasst und/oder dass
c. der Druckgasspeicher (42) mit jedem der Vielzahl von ersten Steuereinheitsauslässen (3) und/oder mit jedem der Vielzahl von zweiten Steuereinheitsauslässen (7) verbindbar oder verbunden ist und/oder dass
d. der Vakuumgasspeicher (44) mit jedem der Vielzahl von ersten Steuereinheitsauslässen (3) verbindbar oder verbunden ist.

12. Fluidsteuerungsvorrichtung (80) nach Anspruch 10 oder 11, **dadurch gekennzeichnet, dass** die Steuereinheit
a. eine Vielzahl von Ventilen (32), wobei jedes der Ventile (32) fluidisch mit einem der Vielzahl von ersten Steuereinheitsauslässen (3) und mit dem Druckgasspeicher (42) und dem Vakuumgasspeicher (44) verbunden ist, und/oder
b. eine Vielzahl von Ventilen (32) umfasst, wobei jedes der Ventile (32) fluidisch mit einem der Vielzahl von zweiten Steuereinheitsauslässen (7) und mit dem Druckgasspeicher (42) verbunden ist.

13. Fluidsteuerungsvorrichtung (80) nach einem der Ansprüche 1 bis 12, **dadurch gekennzeichnet, dass**
a. die Verarbeitungsvorrichtung (4) mindestens einen Behälter (20) zur Aufnahme der Fluidprobe (26) und einen den Behälter (20) abdeckenden Deckel (14) umfasst oder dass
b. die Verarbeitungsvorrichtung (4) mindestens einen Behälter (20) zur Aufnahme der Fluidprobe (26) und einen den Behälter (20) abdeckenden Deckel (14) umfasst, wobei der Deckel (14) mindestens einen in den Behälter (20) ragenden und mit dem ersten Steuereinheitsauslass (3) fluidisch verbundenen Verlängerungsschlauch (24) umfasst.

14. Fluidsteuerungsvorrichtung (80) nach einem der Ansprüche 1 bis 13, **dadurch gekennzeichnet, dass** die Steuerung das Ventil (32) so steuert, dass ein von dem Druckgasspeicher (42) bereitgestellter Überdruck über den Deckel (14) in dem Behälter (20) oder ein von dem Vakuumgasspeicher (44) bereitgestellter Unterdruck über den Deckel (14) in dem Behälter (20) angewendet wird.

15. Fluidsteuerungsvorrichtung (80) nach einem der Ansprüche 1 bis 14, **dadurch gekennzeichnet, dass** die Steuerung das weitere Ventil (62) so steuert, dass in der Kammer (32) der Behandlungsvorrichtung (30) ein vorbestimmter physikalischer Zustand erreicht wird.

## Revendications

1. Dispositif de régulation de fluide (80) comprenant :
au moins un dispositif de traitement (30) ayant une chambre (32) pour recevoir un dispositif de traitement (4),
au moins un dispositif de traitement (4) qui est agencé dans la chambre (32) du dispositif de traitement (30), et une unité de régulation (1) comprenant
une unité de pression (2) pour fournir une pression positive et/ou négative,
au moins une première sortie d'unité de régulation (3) reliée fluidiquement au dispositif de traitement (4) comprenant au moins un réceptacle (20) pour recevoir un échantillon de fluide (26),
au moins une seconde sortie d'unité de régulation (7) reliée fluidiquement au dispositif de traitement (30), et
une unité de liaison (8) au moyen de laquelle l'unité de pression (2) est reliée fluidiquement à la première sortie d'unité de régulation (3) et/ou à la seconde sortie d'unité de régulation (7), dans lequel
l'unité de régulation (1) est adaptée pour réguler le traitement de l'échantillon de fluide (26) dans le dispositif de traitement (4) en appliquant une pression positive ou négative fournie par l'unité de pression (2) à la première sortie d'unité de régulation (3) au moyen de l'unité de liaison (8) et pour réguler un état physique dans la chambre (32) du dispositif de traitement (4).

2. Dispositif de régulation de fluide (80) selon la revendication 1, **caractérisé en ce que** l'unité de régulation (1) est adaptée pour réguler l'état physique dans la chambre (32) du dispositif de traitement en reliant fluidiquement l'unité de pression (2) à la seconde sortie d'unité de régulation (7) ou en les séparant fluidiquement au moyen de l'unité de liaison (8).

3. Dispositif de régulation de fluide (80) selon la revendication 1 ou 2, **caractérisé en ce que** l'unité de pression (2) comprend un réservoir de gaz sous pression (42) ayant une pression positive, dans lequel le réservoir de gaz sous pression (42) peut être relié ou est relié fluidiquement à la première sortie d'unité de régulation (3) et/ou à la seconde sortie d'unité de régulation (7).

4. Dispositif de régulation de fluide (80) selon l'une des revendications 1 à 3, **caractérisé en ce que** l'unité de pression comprend un réservoir de gaz sous vide (44) ayant une pression négative, dans lequel le réservoir de gaz sous vide (44) peut être relié ou est relié fluidiquement à la première sortie d'unité de régulation (3) et/ou à la seconde sortie d'unité de régulation (7).

5. Dispositif de régulation de fluide (80) selon l'une des revendications 1 à 4, **caractérisé en ce que**
a. l'unité de régulation (2) est adaptée pour fournir le même gaz au dispositif de traitement (30) et au dispositif de traitement (4) et/ou **en ce que**
b. le réservoir de gaz sous pression (42) fournit le même gaz à la première sortie d'unité de régulation (3) et à la seconde sortie d'unité de régulation (7).

6. Dispositif de régulation de fluide (80) selon l'une des revendications 3 à 5, **caractérisé en ce que** l'unité de liaison comprend au moins une vanne (34) pour relier fluidiquement de manière sélective le réservoir de gaz sous pression (42) ou le réservoir de gaz sous vide (44) à la première sortie d'unité de régulation (3).

7. - Dispositif de régulation de fluide (80) selon l'une des revendications 3 à 6, **caractérisé en ce que** l'unité de liaison (8) comprend au moins une autre vanne (62) qui, dans une première position, relie fluidiquement le réservoir de gaz sous pression (42) au dispositif de traitement (30) et qui, dans une seconde position, sépare la liaison fluidique entre le réservoir de gaz sous pression (42) et le dispositif de traitement (30).

8. Dispositif de régulation de fluide (80) selon la revendication 6 ou 7, **caractérisé en ce que** l'unité de régulation (1) comprend au moins un dispositif de régulation pour réguler la vanne (34) et/ou l'autre vanne (62).

9. Dispositif de régulation de fluide (80) selon la revendication 8, **caractérisé en ce que**
a. le dispositif de régulation régule la vanne (34) en fonction d'un flux de gaz circulant à travers la première sortie d'unité de régulation (3) et/ou **en ce que**
b. le dispositif de régulation régule la vanne (34) en fonction d'un état physique, en particulier d'une pression, dans le dispositif de traitement (36) et d'un état physique, en particulier d'une pression, du dispositif de traitement (4).

10. Dispositif de régulation de fluide (80) selon l'une des revendications 1 à 9, **caractérisé en ce que** l'unité de régulation comprend
a. une pluralité de premières sorties d'unité de régulation (3), dans lequel chaque sortie de la pluralité de premières sorties d'unité de régulation (3) peut être reliée fluidiquement à un dispositif de traitement (4) et/ou
b. une pluralité de secondes sorties d'unité de régulation (7), dans lequel chaque sortie de la pluralité de secondes sorties d'unité de régulation (7) peut être reliée fluidiquement à un dispositif de traitement (30).

11. Dispositif de régulation de fluide (80) selon la revendication 10, **caractérisé en ce que**
a. l'unité de régulation (1) comprend simplement une unité de pression (2) et/ou **en ce que**
b. l'unité de régulation (1) comprend plusieurs unités de liaison (8) et/ou **en ce que**
c. le réservoir de gaz sous pression (42) peut être relié ou est relié à chaque sortie de la pluralité de premières sorties d'unité de régulation (3) et/ou à chaque sortie de la pluralité de secondes sorties d'unité de régulation (7) et/ou **en ce que**
d. le réservoir de gaz sous vide (44) peut être relié ou est relié à chaque sortie de la pluralité de premières sorties d'unité de régulation (3).

12. Dispositif de régulation de fluide (80) selon la revendication 10 ou 11, **caractérisé en ce que** l'unité de régulation comprend
a. une pluralité de vannes (32), dans lequel chacune des vannes (32) est reliée fluidiquement à une sortie de la pluralité de premières sorties d'unité de régulation (3) et au réservoir de gaz sous pression (42) et au réservoir de gaz sous vide (44) et/ou
b. une pluralité de vannes (32), dans lequel chacune des vannes (32) est reliée fluidiquement à une sortie de la pluralité de secondes sorties d'unité de régulation (7) et au réservoir de gaz sous pression (42).

13. Dispositif de régulation de fluide (80) selon l'une des revendications 1 à 12, **caractérisé en ce que**
a. le dispositif de traitement (4) comprend au moins un réceptacle (20) pour recevoir l'échantillon de fluide (26) et un couvercle (14) recouvrant le réceptacle (20) ou **en ce que**
b. le dispositif de traitement (4) comprend au moins un réceptacle (20) pour recevoir l'échantillon de fluide (26) et un couvercle (14) recouvrant le réceptacle (20), dans lequel le couvercle (14) comprend au moins un tube d'extension (24) s'étendant dans le réceptacle (20) et étant relié fluidiquement à la première sortie d'unité de régulation (3).

14. Dispositif de régulation de fluide (80) selon l'une des revendications 1 à 13, **caractérisé en ce que** le dispositif de régulation régule la vanne (32), de telle sorte qu'une pression positive fournie par le réservoir de gaz sous pression (42) est appliquée par l'intermédiaire du couvercle (14) dans le réceptacle (20) ou qu'une pression négative fournie par le réservoir de gaz sous vide (44) est appliquée par l'intermédiaire du couvercle (14) dans le réceptacle (20).

15. Dispositif de régulation de fluide (80) selon l'une des revendications 1 à 14, **caractérisé en ce que** le dispositif de régulation régule l'autre vanne (62), de telle sorte qu'un état physique prédéterminé est atteint dans la chambre (32) du dispositif de traitement (30).
